# EUROPEAN PATENT APPLICATION

(11) **EP 4 575 481 A1**
(43) Date of publication of application: **25.06.2025**
(21) Application number: 23307373.3
(22) Date of filing: 22.12.2023
(51) Int. Cl.: G01N 27/12, G01N 33/00

(54) **GAS SENSOR COMPRISING A GRAPHENE LAYER**

(71) Applicant: Grapheal, 38000 Grenoble (FR)
(72) Inventor: FOROUGHI, Farshad, 38000 Grenoble (FR); OTHMEN, Riadh, 38000 Grenoble (FR); BOUCHIAT, Vincent, 38000 Grenoble (FR); DJOHARIAN, Behnaz, 38000 Grenoble (FR)
(74) Representative: Cabinet Beaumont

(57) **Abstract**

The present disclosure relates to a gas sensor (100) comprising a layer of graphene (110), at least a first side of the layer of graphene being decorated by metallic nanoparticles (115) and covered by a first polymer layer (120) composed of parylene.

## Description

### Technical field

The present disclosure relates generally to gas sensors and more particularly to gas sensors comprising a graphene layer.

### Background art

The measurement of gas concentrations is essential for certain applications, such as for example to perform breath analysis, measure levels of volatile organic compounds in air or to verify toxicity or potentially explosive levels of chemicals in an environment.

Traditional methods for gas detection, such as gas chromatography and mass spectrometry are hindered by bulky nature of the required apparatus, high maintenance costs, and lengthy sampling and measurement times.

A resistive hydrogen sensor has been proposed that uses a metal oxide semiconductor device. However, this method necessitates high temperatures to operate, posing challenges for low power applications. Furthermore, metal-oxide semiconductor-based resistive hydrogen sensors exhibit some other drawbacks, including suboptimal selectivity and a dependence on oxygen for operation, implying that their operation is affected by varying oxygen concentrations. Other types of solid-state sensors based on an electrochemical reaction with solid electrolytes have recently been proposed. However, they are costly, consume readily high power (above 10 microwatts) and require a three-wire potentiostat to operate.

There is thus a need for a gas sensor having a relatively low cost and/or having relatively low power consumption while maintaining high sensitivity and selectivity with respect to current solutions.

### Summary of Invention

According to one aspect, there is provided a gas sensor comprising a layer of graphene, at least a first side of the layer of graphene being decorated by metallic nanoparticles and covered by a first polymer layer composed of parylene.

According to one embodiment, a second side of the layer of graphene, opposite to the first side, is decorated by metallic nanoparticles and covered by a second polymer layer composed of parylene.

According to one embodiment, the gas sensor further comprises:
- a layer of electrolyte covering a second side of the layer of graphene, opposite to the first side; and
- an electrode in contact with the layer of electrolyte and configured to apply a biasing voltage to the layer of electrolyte.

According to a further aspect, there is provided a gas sensor comprising:
- a layer of graphene, decorated by metallic nanoparticles on one or both sides of the layer of graphene;
- a first polymer layer covering a first side of the layer of graphene; and
- a second polymer layer covering a second side of the layer of graphene, the second side being opposite to the first side, the gas sensor being configured to detect gas penetrating the polymer layers on the first and/or second sides of the layer of graphene.

According to one embodiment, the gas sensor is configured to detect hydrogen.

According to one embodiment, the metallic nanoparticles comprise nanoparticles of one or more of the materials in the following list: silver; platinum; palladium; or an alloy of these materials.

According to one embodiment, the first polymer layer has a first side in contact with the graphene layer, the gas sensor further comprising a metal layer covering a second side of the polymer layer opposite to the first side of the polymer layer.

According to a further aspect, there is provided an electronic device, comprising:
- a gas sensor comprising a layer of graphene coupled to first and second electrodes;
- an electronic circuit comprising: a biasing circuit configured to bias the graphene layer by applying a voltage or a current to the first electrode; and an analog-to-digital converter configured to convert a voltage between the first electrode and the second electrode into a digital value; and
- a transmission circuit configured to wirelessly transmit the digital value via an antenna of the electronic device.

According to one embodiment, the electronic circuit is configured to be powered by energy harvesting of a received RF signal.

According to one embodiment, the gas sensor comprised in the electronic circuit is implemented by the gas sensor described above.

According to a further aspect, there is provided a system comprising:
- the electronic device described above; and
- a membrane attached to the electronic device and enclosing the gas sensor of the electronic device, the membrane being suitable for being pierced by a straw for supplying a gas to the gas sensor.

According to a further aspect, there is provided a system comprising:
- a first compartment comprising an inlet; and
- the electronic device described above, located inside the second compartment.

According to one embodiment, the system further comprises: an input valve at the interface between the first compartment and a second compartment; and an output valve on one side of the second compartment.

According to a further aspect, there is provided a method of fabrication of a gas sensor, the method comprising:
- the deposition of metallic nanoparticles on a layer of graphene; and
- the deposition of a first polymer layer composed of parylene covering the graphene layer decorated with metallic nanoparticles.

According to a further aspect, there is provided a method of fabrication of a gas sensor, the method comprising:
- the deposition of metallic nanoparticles on a first side of a layer of graphene;
- forming a first electrode in contact with the layer of graphene, for example by printing the first electrode in a first region of the first side of the layer of graphene using conductive ink;
- the deposition of a first polymer layer covering the first side of the layer of graphene;
- the deposition of metallic nanoparticles on a second side of the layer of graphene, the second side being opposite to the first side;
- forming a second electrode in contact with the layer of graphene, for example by printing the second electrode in a second region of the second side of the layer of graphene using conductive ink; and
- the deposition of a second polymer layer covering the second side of the layer of graphene.

### Brief description of drawings

The foregoing features and advantages, as well as others, will be described in detail in the following description of specific embodiments given by way of illustration and not limitation with reference to the accompanying drawings, in which:
Figure 1 is a cross-section view of a gas sensor according to an example embodiment of the present disclosure;
Figure 2 shows cross-section views of the gas sensor of Figure 1 during successive steps of a fabrication process according to an example embodiment of the present disclosure;
Figure 3 is a cross-section view of a gas sensor according to a further example embodiment of the present disclosure;
Figure 4 shows cross-section views of the gas sensor of Figure 3 during successive steps of a fabrication process according to an example embodiment of the present disclosure;
Figure 5 is a cross-section view of a gas sensor according to yet another example embodiment of the present disclosure;
Figure 6A shows cross-section views of the gas sensor of Figure 1 during successive steps of a fabrication process to accommodate for the connection of two electrodes to the gas sensor, according to one embodiment;
Figure 6B shows cross-section views of the gas sensor of Figure 1 during successive steps of a fabrication process to accommodate for the connection of two electrodes to the gas sensor, according to another embodiment;
Figure 6C is a cross-section view of the gas sensor of Figure 3 with electrodes;
Figure 7A schematically illustrates an electronic circuit for measuring the conductivity of a graphene layer of the gas sensor of Figure 1, Figure 3 or Figure 5 according to an example embodiment of the present disclosure;
Figure 7B schematically illustrates an electronic circuit for measuring the conductivity of a graphene layer of the gas sensor of Figure 1, Figure 3 or Figure 5 according to another example embodiment of the present disclosure;
Figure 8 schematically illustrates an electronic device according to an example embodiment of the present disclosure;
Figure 9 is a graph illustrating a relative change in resistance over time of the graphene layer of the hydrogen gas sensor of Figure 1 for different hydrogen concentrations;
Figure 10 is a cross-section view schematically illustrating a system for gas detection according to an example embodiment of the present disclosure; and
Figure 11 is a perspective view of a system for gas detection according to a further example embodiment of the present disclosure.

### Description of embodiments

Like features have been designated by like references in the various figures. In particular, the structural and/or functional features that are common among the various embodiments may have the same references and may dispose identical structural, dimensional and material properties.

For the sake of clarity, only the operations and elements that are useful for an understanding of the embodiments described herein have been illustrated and described in detail.

Unless indicated otherwise, when reference is made to two elements connected together, this signifies a direct connection without any intermediate elements other than conductors, and when reference is made to two elements coupled together, this signifies that these two elements can be connected or they can be coupled via one or more other elements.

In the following disclosure, unless indicated otherwise, when reference is made to absolute positional qualifiers, such as the terms "front", "back", "top", "bottom", "left", "right", etc., or to relative positional qualifiers, such as the terms "above", "below", "higher", "lower", etc., or to qualifiers of orientation, such as "horizontal", "vertical", etc., reference is made to the orientation shown in the figures.

Unless specified otherwise, the expressions "around", "approximately", "substantially" and "in the order of" signify within 10 %, and preferably within 5 %.

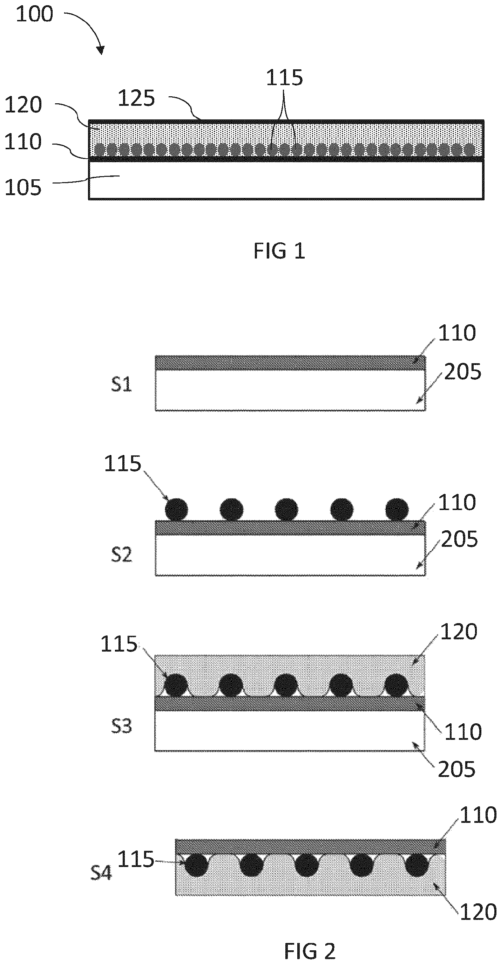

Figure 1 is a cross-section view of a gas sensor 100 according to an example embodiment of the present disclosure. In some embodiments, the gas sensor 100 is a hydrogen gas sensor configured to detect the presence of dihydrogen gas in the environment of the sensor 100.

The gas sensor 100 comprises a substrate 105 configured to mechanically support the detection layers of the gas sensor. The substrate 105 is for example a layer of polymer, for example of polyethylene terephthalate (PET) or poly(para-xylylene) also referred to as n-xylylene or parylene. In the case of parylene, the substrate 105 is for example more specifically composed of parylene-C, parylene-N, parylene-D or a mixture of these compounds.

The substrate 105 of the gas sensor 100 is covered by a layer of graphene 110. In some embodiments, the graphene layer 110 is a graphene monolayer, that is a layer of graphene having a thickness of only one atom. According to another embodiment, the graphene layer 110 is a multilayer comprising at least two layers of graphene. The layer of graphene 110 is electrically conductive.

The top side of the graphene layer 110 is partially covered by metallic nanoparticles 115. The metallic nanoparticles 115 are for example composed of palladium, platinum, silver or an alloy of these elements. In one embodiment, and particularly in the case of a hydrogen gas sensor, the metallic nanoparticles 115 are of palladium or platinum. The diameter of the metallic nanoparticles 115 is for example lower than 5 nanometers and is for example comprised between 1 and 2 nanometers. The metallic nanoparticles 115 are in direct contact with the graphene layer 110. The conductivity of the graphene layer 110 is modified by an interaction between molecules of the gas to be detected with the metallic nanoparticles. According to one embodiment, in the case of a hydrogen gas sensor, the metallic nanoparticles 115 absorb dihydrogen molecules located in their vicinity. The absorption of dihydrogen molecules by the metallic nanoparticles 115 affect the electronic interaction of the metallic nanoparticles 115 with graphene and in turn tune the conductivity of the graphene layer 110 by field effect. The conductivity of the graphene layer 110 thus varies as a function of the concentration of dihydrogen surrounding the metallic nanoparticles and the dihydrogen gas sensor 100 acts as a chemoresistance, that is a variable resistance for which the resistance value is a function of the concentration of dihydrogen. A similar effect can be achieved for other gases to be detected.

The graphene layer 110 and the metallic nanoparticles 115 are further covered by a layer of polymer 120. In the case of a hydrogen gas sensor, the layer of polymer 120 is for example a layer of parylene, for example parylene-C, parylene-N, parylene-D or a mixture of these compounds. Parylene is a porous material and its pores are large enough to allow dihydrogen molecules to pass through it, but small enough to block other larger molecules from passing through. Depending, for example, on its precise composition and thickness, the polymer layer 120 thus forms a molecular sieve in the gas sensor 100, thereby aiding the gas selectivity of the sensor. In the case of a hydrogen gas sensor having a polymer layer 120 of parylene, this parylene layer 120 for example has a thickness of between 100 nm and 10 µm.

According to one embodiment, a layer of metal 125 is deposited on the surface of the polymer layer 120. The metal is for example palladium, platinum, silver or a compound of these elements. The layer of metal 125 is for example configured to filter molecules reaching the gas sensor 100 before they reach the surface of the polymer layer 120, and thereby further increases the selectivity of the gas sensor 100. In the case of a hydrogen gas sensor, the metal layer 125 for example has a thickness of between 0.5 nm and 50 nm.

The composition of the polymer layer 120, its thickness and the addition of the metal layer 125 at the surface of the polymer layer 120 are for example used to tune the sensitivity and enhance the selectivity of the gas sensor 100 to a specific gas.

Figure 2 shows cross-section views of the hydrogen gas sensor 100 of Figure 1 during successive steps of a fabrication process according to an example embodiment of the present disclosure.

During a process step S1, the graphene layer 110 is grown on a temporary substrate layer 205, for example made of copper, using chemical vapor deposition.

During a process step S2 following the step S1, the metallic nanoparticles 115 are deposited on the surface of the graphene layer 110 to partially cover it, for example using physical vapor deposition in vacuum or electrochemical reduction of a metal salt in water. For example, at least 5% of the surface of the graphene layer 110 is left uncovered during the step S2 and for example at least 10% of the surface of the graphene layer 110 is left uncovered during the step S2.

During a process step S3 following the step S2, the layer of polymer 120 is deposited, for example using chemical vapor deposition. The thickness of the layer of polymer 120 is for example adjusted according to the gas that is to be detected.

While not illustrated in Figure 2, according to one embodiment, the layer of metal 125 of Figure 1 is deposited at the surface of the layer of polymer 120 after the process step S3. For example, a layer of palladium is deposited using physical vapor deposition or electrochemistry.

During a process step S4 following the step S3, the temporary substrate layer 205 is for example removed by chemical attack with ferric chloride or sodium persulfate, or by delamination by oxidation of copper in hot water, or by delamination by electrochemical action and formation of a gas film in the copper/graphene interface.

During a process step following the step S4, not represented in Figure 2, the substrate layer 105 of Figure 1 is for example deposited on the graphene layer 110 in place of the temporary substrate layer 205, in order to obtain the gas sensor 100 of Figure 1. According to one embodiment, the substrate layer 105 is of parylene and is deposited using chemical vapor deposition, in a similar fashion to the process step S3. According to another embodiment, the substrate layer 105 contain an additional film of PET covered with ethylenevinyl acetate (EVA) and is for example deposited using hot lamination, either directly on the layer of graphene 110 or directly on the substrate layer 105, taking advantage of the hot melt properties of EVA. The substrate layer 105 protects the graphene layer and provides support for the gas sensor 100.

Some of the method steps of Figure 2 are described in more detail in the patent publications FR3131076A1 and WO2023118723, in the name of the present applicant, and the contents of those patent publications are hereby incorporated by reference in their entirety to the extent permitted by the law.

While in the embodiment of Figure 2, the substrate is the temporary substrate layer 205, in alternative embodiments, the substrate layer may be changed for a layer of another material prior to the steps S2 to S4. In such a case, after the step S1, a substrate layer of polymer, for example composed of parylene, is deposited on the graphene layer 110, and the temporary substrate layer 205 is removed as described in step S4. The metallic nanoparticles 115 are then deposited according to step S2 on the bare surface of the graphene layer 110. The layer of parylene 120 is then deposited on top of the metallic nanoparticles 115 according to the step S3.

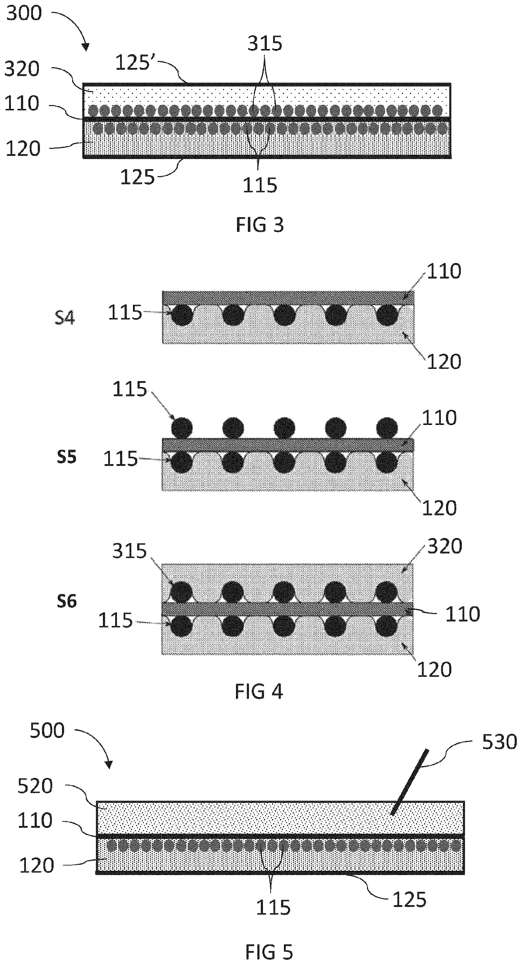

Figure 3 is a cross-section view of a gas sensor 300 according to a further example embodiment of the present disclosure.

The gas sensor 300 of Figure 3 does not comprise the substrate 105 of Figure 1. The gas sensor 300 comprises the metallic nanoparticles 115 and the first polymer layer 120 on one side of the graphene layer 110 and comprises metallic nanoparticles 315 and a second polymer layer 320 on the opposite side of the graphene layer 110.

The metallic nanoparticles 315 are for example composed of palladium, platinum, silver or a compound of these elements. The metallic nanoparticles 115 and 315 have for example the same composition as each other. In alternative embodiments, the metallic nanoparticles 115 and 315 have different compositions.

The diameter of the metallic nanoparticles 315 is for example of less than 5 nanometers and is for example comprised between 1 and 2 nanometers.

The metallic nanoparticles 315 are in direct contact with the layer of graphene 110.

According to one embodiment, the metallic nanoparticles 315 are used for gas detection. According to another embodiment, the metallic nanoparticles 315 are used as doping agents to dope the polymer layer 320 and set the electric potential of the graphene layer 110 in order to tune the gas sensor 300 to operate at a relatively high sensitivity.

In an alternative embodiment, chemical components, not illustrated in Figure 3, are mixed in the polymer layer 320 to dope the polymer layer 320 and tune the electric potential of the graphene layer 110 to tune the gas sensor 300 to operate at a relatively high maximum sensitivity.

The layers of polymer 120 and 320 are for example a layer of parylene, for example parylene-C, parylene-N, parylene-D or a mixture of these compounds, or any other suitable polymer. The polymer layers 120 and 320 have for example the same composition as each other. In alternative embodiments, the layers 120 and 320 have different compositions. The layer 120 or the layer 320 for example constitutes the substrate of the gas sensor 300.

The polymer layers 120 and 320 are for example porous layers that act as a polymeric molecular sieve membrane configured to filter particles small enough to get through one layer of polymer 120, 320 and reach the metallic nanoparticles 115, 315. The metallic nanoparticles 115, 315 are chosen so that their conductivity can be modulated according the concentration of a specific gas. The modulation of the conductivity of the metallic nanoparticles 115, 315 leads to a modulation of the conductivity of the graphene layer they are in contact with. Hence, the device 300 can be used as a gas sensor, for example for dihydrogen, ammoniac, ethanol, methane, etc.

The composition of the polymer layers 120, 320, their thickness and the addition of the metal layers 125, 125' at the surface of the polymer layers 120, 320 are for example used to tune the sensitivity and selectivity of the gas sensor 300 to a specific gas.

One advantage of having the metallic nanoparticles 115, 315 and the polymer layers 120, 320 on both sides of the graphene layer 110 is that it increases the surface area over which gas can be detected, and thus increases the sensitivity of the gas sensor 300.

Figure 4 shows cross-section views of the gas sensor 300 of Figure 3 during successive steps of a fabrication process according to an example embodiment of the present disclosure.

The device illustrated in a first step S4 of Figure 4 is for example obtained by carrying out the steps S1 to S4 of Figure 2. The graphene layer 110 is partially covered by the metallic nanoparticles 115 on a first side of the layer of graphene 110 and the layer of polymer 120 has been deposited on the first side of the layer of graphene 110. The temporary substrate layer 205 has been removed, leaving the second side of the graphene layer 110 uncovered.

During a process step S5 of Figure 4 following the step S4 of Figure 2, the metallic nanoparticles 315 are deposited on the surface of the second side of the graphene layer 110 to partially cover it, for example in a similar fashion to the step S2 of Figure 2, for example using physical vapor deposition or electrochemistry. For example, at least 5% of the surface of the second side of the graphene layer is left uncovered during the step S5 and for example at least 10% of the surface of the second side of the graphene layer is left uncovered during the step S5.

During a process step S6 following the step S5, the layer of polymer 320 is for example deposited on the second side of the graphene layer 110 in order to obtain the gas sensor 300 of Figure 3. For example, the polymer layer 320 is deposited according to a process similar to that of step S3 of Figure 2, for example using chemical vapor deposition.

While not illustrated in Figure 4, according to one embodiment, the layer of metal 125 of Figure 3 is deposited at the surface of the layer of polymer 120 after the process step S3 and the layer of metal 125' is deposited at the surface of the layer of polymer 320 after the process step S6.

Figure 5 is a cross-section view of a gas sensor 500 according to yet another example embodiment of the present disclosure.

The gas sensor 500 comprises the metallic nanoparticles 115 and the polymer layer 120 on one side of the graphene layer 110. According to one embodiment, a layer of metal 125 is present at the surface of the layer of polymer 120.

The second side of the graphene layer 110 is covered by a layer of electrolyte 520 that is in contact with the graphene layer 110. The layer of electrolyte 520 is for example a liquid, such as a gel, or a solid. The electrolyte 520 is for example a solid polymer electrolyte, for example made of polyvinyl acetate (PVA) and magnesium perchlorate.

An electrode 530 is in contact with the electrolyte 520 and is configured to bias the electrolyte 520, for example with a fixed voltage. Applying a bias voltage to the electrolyte layer 520 biases the graphene layer 110 that it is in contact with, and thereby allows to set the working point of the gas sensor 500.

The gas sensor 500 is for example obtained from the process steps S1 to S4 of Figure 1 followed by the deposition of a layer of electrolyte 520 on the exposed surface of the graphene layer 110. In some embodiments, and in particular in the case that the electrolyte is a liquid, it is for example sealed within a casing (not illustrated) such that it is trapped between the casing and the graphene layer 110. In some embodiments, for example if the electrolyte is a solid, a substrate polymer layer is deposited at the surface of the layer of electrolyte 520. For example, the substrate polymer layer comprises a layer of parylene, of PET or of PET covered with EVA.

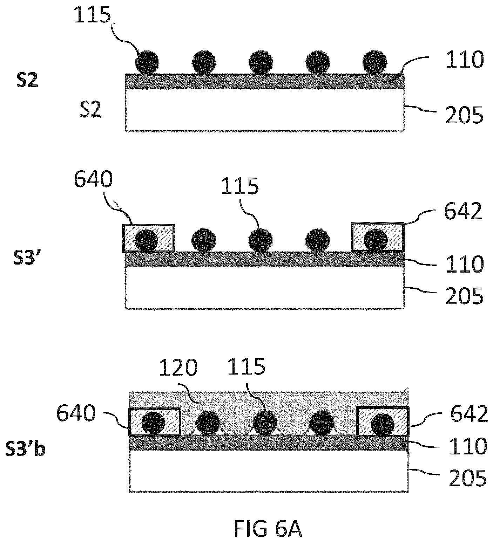

Figure 6A shows cross-section views of the gas sensor 100, 300, 500 of Figure 1 during successive steps of a fabrication process to accommodate for the connection of two electrodes 640, 642 to the gas sensor, according to one embodiment.

The measurement of the concentration of a gas using the gas sensor 100, 300, 500 is for example performed by connecting both edges of the graphene layer 110 to the electrodes 640, 642 in order to bias the graphene layer 110 and/or to make measurements.

For example, the process step S3 of Figure 2 is modified in order to integrate steps of depositing the electrodes 640, 642.

During a process step S3' following the step S2 of Figure 2, the electrodes 640, 642 are deposited on the graphene layer 110 partially covered by metallic nanoparticles, for example by ink printing using a conductive ink, for example using an ink comprising silver particles.

During a process step S3'b following the step S3', the polymer layer 120 is deposited according to the process step S3 of Figure 2 on the surface of the graphene layer 110 and on the surface of the electrodes 640, 642. The polymer layer 120 protects the electrodes 640, 642 and/or the metallic nanoparticles 115 from corrosion, for example particular in the case that the polymer layer 120 is formed of parylene, and particularly for electrodes and/or metallic nanoparticles 115 made of silver.

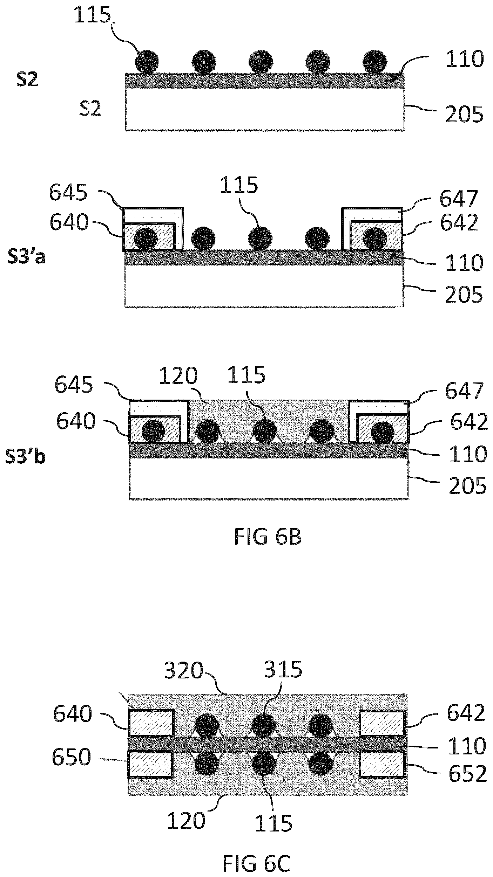

Figure 6B shows cross-section views of the gas sensor 100, 300, 500 of Figure 1 during successive steps of a fabrication process to accommodate for the connection of two electrodes 640, 642 to the gas sensor, according to another embodiment.

In a preferred embodiment, in a process step S3'a following the deposition of the metallic nanoparticles 115 described in the process step S2 of Figure 2, the electrodes 640, 642 are ink printed using a conductive ink, for example using an ink comprising silver particles, onto a sheet of PET covered with EVA, on the EVA. The sheet of PET covered with EVA comprises openings, for example in the region between the electrodes where the polymer layer 120 will be deposited. The electrodes 640, 642 and the PET layer 645, 647 are for example deposited on the surface of the graphene layer 110, for example using a hot melt lamination process. The hot melt lamination process step is for example performed according to the process detailed in the European patent application EP23305773 filed on 15 May 2023, in the name of the present applicant, the contents of this patent application being hereby incorporated by reference in its entirety to the extent permitted by the law.

During a process step S3'b following the step S3'a, the polymer layer 120 is deposited according to the process step S3 of Figure 2 on the surface of the graphene layer 110, in the openings of the sheet of PET.

The method of Figure 6B can for example be adapted in order to for the electrodes 640, 642 for contacting the graphene layer 110 in the gas sensor 300 of Figure 3. In such a case, the step S2 is for example applied to the structure of step S4 of Figure 2 in order to deposit the metallic nanoparticles 315 on the exposed surface of the graphene layer 110. The steps S3'a and S3'b are then for example performed as described above.

An alternative method of forming electrodes to contact the graphene layer 110 in the gas sensor 300 of Figure 3 will now be described with reference to Figure 6C.

Figure 6C is a cross-section view of the gas sensor of Figure 3 with electrodes 640, 642, 650, 652.

Similarly to the connection of the two electrodes 640, 642 on one side of the graphene layer 100 detailed in Figure 6A, two further electrodes 650, 652 are for example deposited on the other side of the graphene layer 110 in the gas sensor 300 of Figure 3.

According to one embodiment, in order to form these electrodes, the steps S3' and S3'b of Figure 6A are for example replicated in place of the step S6 of Figure 4.

The electrodes 640 and 650 at one edge of the graphene layer 110 and the electrodes 642, 652 at the other edge of the graphene layer 110 are for example connected to each other to form two contacts, one on each edge of the graphene layer 110.

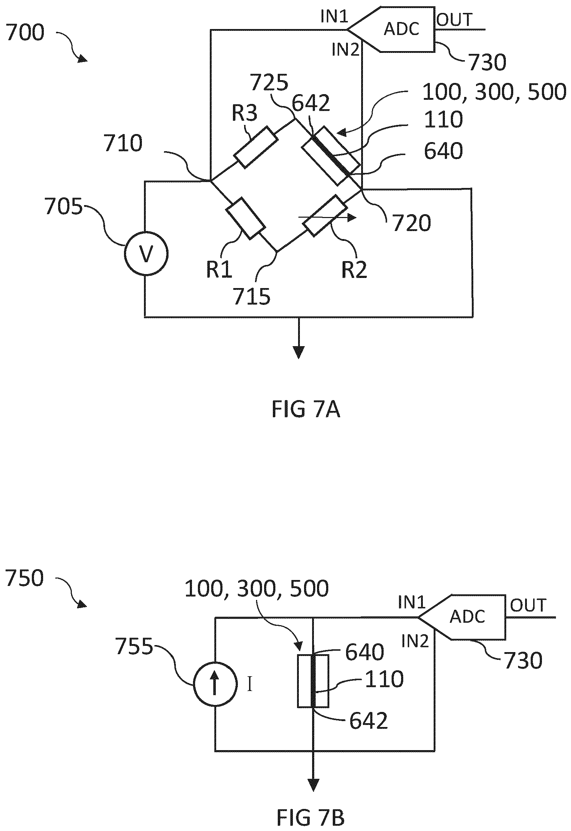

Figure 7A schematically illustrates an electronic circuit 700 for measuring the conductivity of a graphene layer of the gas sensor 100, 300 or 500 of Figure 1, Figure 3 or Figure 5 according to an example embodiment of the present disclosure.

The electronic circuit 700 comprises any one of the gas sensors 100, 300, 500 of Figure 1, Figure 3 and Figure 5 respectively that comprises the graphene layer 110 connected between the two electrodes, such as the electrodes 640, 642 of Figure 6A. A voltage source 705 is coupled between the ground of the electronic circuit 700 and a first node 710 of a Wheatstone bridge.

The Wheatstone bridge comprises a first resistance R1 with a fixed resistance value, coupled between the node 710 and a second node 715. The Wheatstone bridge also comprises a second resistance R2 with a variable resistance value, coupled between the node 715 and a third node 720. The Wheatstone bridge further comprises a third resistance R3 with a fixed resistance value, coupled between the node 710 and a fourth node 725.The Wheatstone bridge further comprises the gas sensors 100, 300, 500, connected between the nodes 725 and 720.

An analog-to-digital converter (ADC) 730 has for example two input nodes IN1, IN2 coupled to the nodes 710 and 720 of the Wheatstone bridge and is configured to measure the voltage between these nodes and to convert the voltage into a digital output value at its output node OUT.

By adjusting the value of the resistance R2 and measuring the voltage between the nodes 710 and 720, the value of the resistance of the gas sensor 100, 300, 500 can be deduced.

The graphene layer 110 is biased at a fixed voltage. As the conductivity of the graphene layer 110 varies with the concentration of the surrounding gas, the current flowing through the graphene layer 110 varies as a function of the concentration of surrounding gas. According to one embodiment, the resistance of the gas sensor 100, 300, 500 is comprised between 1 and 5 kilo-ohms, and advantageously the energy consumption of the electronic circuit 700 is in the sub-microwatts regime.

Figure 7B schematically illustrates an electronic circuit 750 for measuring the conductivity of the graphene layer 110 of the gas sensor of Figure 1, Figure 3 or Figure 5 according to another example embodiment of the present disclosure, the circuit 750 for example providing an alternative to the circuit 700 of Figure 7A.

The electronic circuit 750 comprises any one of the gas sensors 100, 300, 500 of Figure 1, Figure 3 and Figure 5 respectively that comprises the graphene layer 110 connected between the two electrodes, such as the electrodes 640, 642 of Figure 6A. A constant current source 755, for example implemented by a galvanostat, is coupled between the ground of the electronic circuit 750 and the first electrode 640 of the gas sensor 100, 300, 500 and is configured to deliver a constant current to the gas sensor. In some embodiments, the level of the constant current generated by the current source 755 is adjustable.

The second electrode 642 of the gas sensor 100, 300, 500 is coupled to the ground of the electronic circuit 750.

An analog-to-digital converter (ADC) 730 has for example two input nodes IN1, IN2 respectively coupled to the first electrode 640 and the second electrode 642 of the gas sensor 100, 300, 500 and is configured to measure the voltage across the gas sensor and to convert the voltage into a digital output value at its output node OUT.

The constant current, for example having a known value, is driven through the gas sensor 100, 300, 500 by the current source 755, and the resistance of the gas sensor 100, 300, 500 is for example deduced from the voltage measured between its electrodes 640, 642.

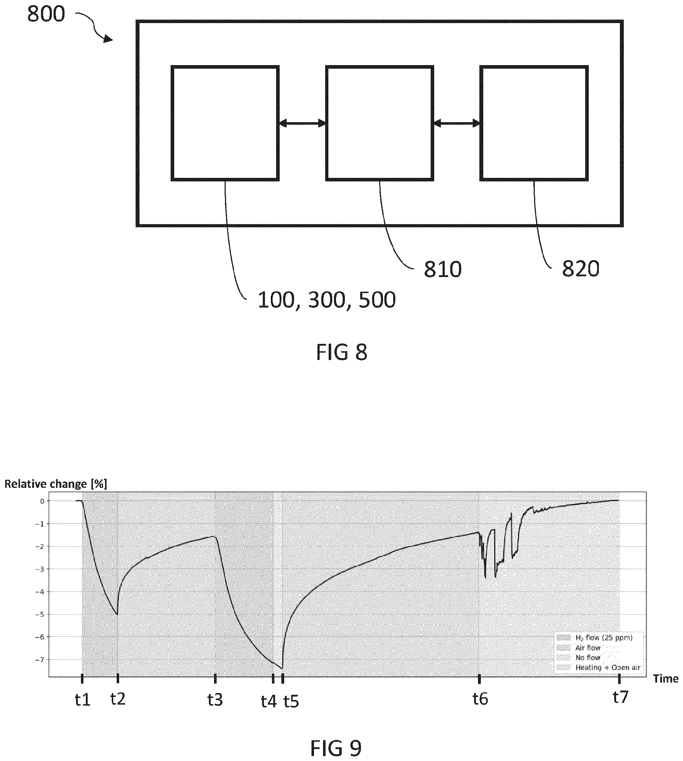

Figure 8 schematically illustrates an electronic device 800 according to an example embodiment of the present disclosure.

The electronic device 800 comprises any one of the gas sensors 100, 300, 500 of Figure 1, Figure 3 and Figure 5 connected to an electronic circuit 810. The electronic circuit 810 is configured to communicate, for example via NFC communications ("NFC Com"), with an external device, represented by a mobile phone or smart phone in the example of Figure 8, via an antenna 820. Alternatively, the external device could be another type of electronics devices, such as a laptop or tablet computer.

The electronic circuit 810 for example comprises the electronic circuit 700 of Figure 7A or the electronic circuit 750 of Figure 7B configured to evaluate the concentration of a specific gas in the environment of the gas sensor 100, 300, 500.

According to one embodiment, the antenna 820 is configured to transmit and receive data wirelessly at a frequency comprised in the range 13 MHz to 14 MHz, which corresponds to the Near Field Communication (NFC) frequency range. For example, the electronic circuit used for the evaluation of the concentration of the specific gas is the electronic circuit 750 of Figure 7B.

According to another embodiment, the antenna 820 is configured to transmit and receive data wirelessly at a frequency comprised between 300 MHz and 3 GHz, which corresponds to the ultra-high frequency (UHF) radio frequency identification (RFID) frequency range. For example, the electronic circuit used for the evaluation of the concentration of the specific gas is the electronic circuit 700 of Figure 7A.

According to one embodiment, the electronic circuit 810 is configured to perform energy harvesting via the antenna 820 to power the electronic device 800, according to a standard protocol known by a person skilled in the art. One advantage of a gas sensor 100, 300, 500 comprising a graphene layer is that it consumes sufficiently small amounts of energy that it can be operated using energy harvesting alone, without the need of another power source such as a battery.

Figure 9 is a graph illustrating a relative change in resistance over time of the graphene layer of the hydrogen gas sensor 100 of Figure 1 for different hydrogen concentrations with respect to an initial condition where the gas sensor 100 is surrounded by air.

The relative change in resistance of the hydrogen gas sensor 100 is for example measured using the electronic circuit 700 of Figure 7A or the electronic circuit 750 of Figure 7B and the electronic device 800 of Figure 8. The relative change is for example measured as a voltage variation.

During the first time period, between t1 and t2, where t1 is 44 seconds and t2 is 5 minutes and 11 seconds, a flux of dihydrogen with a concentration of 25 parts per million (ppm) ("H₂ flow (25ppm)") is sent in the direction of the gas sensor 100. The dihydrogen concentration increases in the vicinity of the gas sensor 100 and a 5% drop is measured by the gas sensor.

During the second time period, between t2 and t3, where t3 is 17 minutes and 25 seconds, a flux of air ("Air flow") is sent in the direction of the gas sensor 100. The dihydrogen concentration decreases in the vicinity of the gas sensor 100 and a gain of about 3.3% is measured by the gas sensor to reach a relative change of about -1.7%.

During time period between t3 and t4, where t4 is about 25 minutes, a flux of dihydrogen ("H₂ flow (25ppm)") with a concentration of 25ppm is sent in the direction of the gas sensor 100. The dihydrogen concentration increases in the vicinity of the gas sensor 100 and a drop of about 5.5% is measured by the gas sensor to reach a relative change of about -7.2%.

During the time period between t4 and t5, where t5 is 25 minutes and 50 seconds, the flux of dihydrogen ("No flow") is stopped and the concentration of dihydrogen stays stable. The voltage drop continues with a similar decay rate to reach a relative change of about -7.5%.

During the time period between t5 and t6, where t6 is 50 minutes and 25 seconds, a flux of air ("Air flow") is sent in the direction of the gas sensor 100. The dihydrogen concentration decreases in the vicinity of the gas sensor 100 and a gain of about 6.1% is measured by the gas sensor to reach a relative change of about -1.4%.

During the last time period, between t6 and t7, where t7 is one hour and 8 minutes, gas flux are stopped and a heating system is switched on ("Heating + Open air"). The heating system causes air movement and sudden variations are measured by the gas sensor 100. The relative change reaches 0 at t7 as the gas surrounding the sensor 100 has a similar composition as in the initial condition at t1.

The gas sensor 100 is able to detect variation of dihydrogen concentration below 25ppm and for example has a 5ppm threshold of detection.

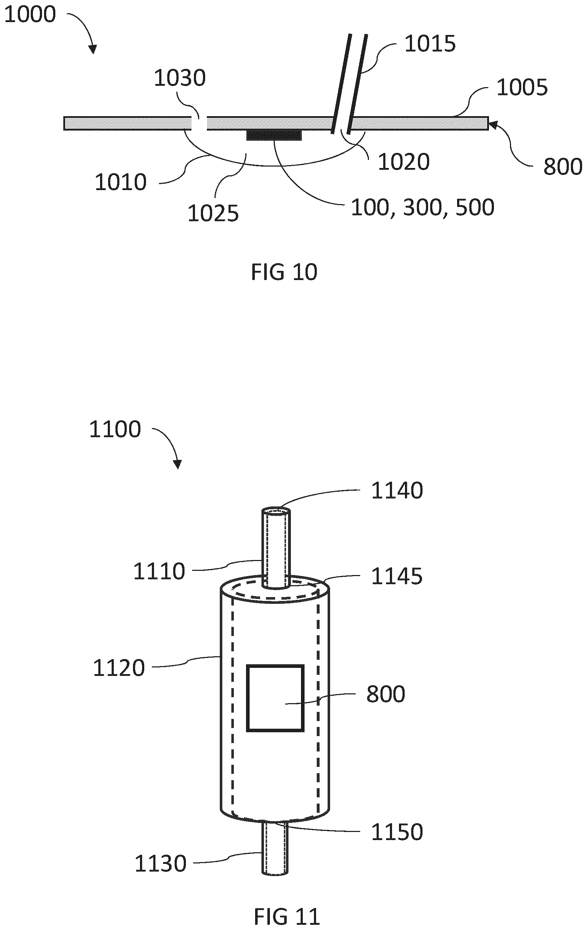

Figure 10 is a cross-section view schematically illustrating a system 1000 for gas detection according to an example embodiment of the present disclosure.

The system 1000 comprises the electronic device 800 of Figure 8 that comprises the gas sensor 100, 300 or 500 of Figures 1, 3 and 5. The system 1000 for example further comprises a membrane 1010 attached to the electronic device 800 and forming a blister enclosing the gas sensor 100, 300, 500 of the electronic device 800.

According to one embodiment, a user blows air into a cavity 1025 defined by the membrane 1010 with a straw 1015, such that the air passes over the gas sensor 100, 300, 500. The gas sensor is for example used to measure the concentration of a given gas, such as hydrogen, ethanol, methane, ammonia, etc., in the user's breath. In some embodiments, the electronic device 800 comprises the gas sensor 100, 300, 500 fixed facing down to the bottom side of a substrate 1005, such as a PCB (printed circuit board) or inlay. An inlet 1020 passes through the substrate 1005 and communicates with the cavity 1025.The inlet 1020 is for example connected to a straw 1015 allowing air to be blown in the cavity 1025. An outlet 1030 is for example also provided, passing through the substrate 1005 and communicating with the cavity 1025, allowing air to leave the cavity 1025.

An advantage of providing the cavity 1025 containing the gas sensor 100, 300, 500 on the underside of the substrate 1005 is that this allows a better detection of certain gases that are lighter than air, for example hydrogen. Indeed, the lighter gas will accumulate at the time of the cavity 1025 where the gas sensor is positioned. Furthermore, water condensation forming in the cavity 1025 and resulting from the moisture in the user's breath will fall to the bottom of the cavity 1025, away from the gas sensor.

The concentration of hydrogen in the user's breath can for example be used to diagnose digestive conditions.

Figure 11 is a perspective view of a system 1100 for gas detection according to a further example embodiment of the present disclosure.

The system 1100 comprises a first compartment 1110 and a second compartment 1120 configured to contain and guide air. According to one embodiment, the system 1100 comprises a third compartment 1130, in contact with the second compartment 1120 and configured to guide air.

The first compartment 1110 comprises an inlet 1140 where a gas to be analyzed enters. For example, the compartment 1110 is a cylinder with an inner cavity and a user can blow into it for their breath to be analyzed. The shape of the first compartment 1110 is not restricted to having a circular cross-section, but could have other forms, such as a rectangular cross-section.

In some embodiments, an input valve 1145 is positioned at the interface between the first 1110 and second 1120 compartments. The input valve 1145 is configured to let a gas in if its velocity is high enough to open the valve and to contain the gas in the second compartment 1120 during the analysis. In other embodiments, there is no valve at the interface between the first 1110 and second 1120 compartments.

In some embodiments, an output valve 1150 is positioned on a wall of the second 1120 compartment. The output valve 1150 is configured to let a gas out of the first compartment 1110 if its velocity is high enough to open the valve and to maintain the gas in the second compartment 1120 during the analysis. In other embodiments, there is no output valve.

According to one embodiment, as a user blows air inside the first compartment 1110 using the inlet 1140, the input 1145 and output 1150 valves open and the gas previously inside the second compartment 1120 is blown out and replaced by the user's breath.

A third compartment 1130 is for example in contact with the second compartment 1120 at the position of the output valve 1150.

The electronic device 800 of Figure 8 is fixed inside the second compartment. The gas sensor of the electronic device 800 analyses the concentration of a gas inside the compartment 1120 over time and the data can be transmitted to an external device, not represented, via the antenna 820.

An advantage of the embodiments described herein is that a gas sensor having relatively low power consumption and relatively low cost can be obtained.

An advantage of printing the electrodes of the graphene layer 110 using a conductive ink is that this can avoid the need for a specific metal deposition step and to have a process compatible with roll-to-roll manufacturing.

Various embodiments and variants have been described. Those skilled in the art will understand that certain features of these embodiments can be combined and other variants will readily occur to those skilled in the art.

Finally, the practical implementation of the embodiments and variants described herein is within the capabilities of those skilled in the art based on the functional description provided hereinabove. In particular, a gas sensor to measure the concentration of dihydrogen has been described but the gas sensor is also suitable for hydrogen. Although the present description details the fabrication of a single gas sensor, it will be apparent to those skilled in the art that several gas sensors can be used to deduce the concentration of a gas based on a linear combination of the responses of the several gas sensors.

## Claims

1. A gas sensor (100, 300, 500) comprising a layer of graphene (110), at least a first side of the layer of graphene being decorated by metallic nanoparticles (115) and covered by a first polymer layer (120) composed of parylene.

2. The gas sensor of claim 1, wherein a second side of the layer of graphene, opposite to the first side, is decorated by metallic nanoparticles (315) and covered by a second polymer layer (320) composed of parylene.

3. The gas sensor according to claim 1, further comprising:
- a layer of electrolyte (520) covering a second side of the layer of graphene, opposite to the first side; and
- an electrode (530) in contact with the layer of electrolyte and configured to apply a biasing voltage to the layer of electrolyte.

4. A gas sensor (300) comprising:
- a layer of graphene (110), decorated by metallic nanoparticles (115, 315) on one or both sides of the layer of graphene;
- a first polymer layer (120) covering a first side of the layer of graphene; and
- a second polymer layer (320) covering a second side of the layer of graphene, the second side being opposite to the first side, the gas sensor being configured to detect gas penetrating the polymer layers on the first and/or second sides of the layer of graphene.

5. The gas sensor of any of claims 1 to 4, configured to detect hydrogen.

6. The gas sensor according to any one of claims 1 to 5, wherein the metallic nanoparticles (115, 315) comprise nanoparticles of one or more of the materials in the following list:
- silver;
- platinum;
- palladium;
or an alloy of these materials.

7. The gas sensor according to any one of claims 1 to 6, wherein the first polymer layer (120) has a first side in contact with the graphene layer, the gas sensor further comprising a metal layer (125) covering a second side of the polymer layer opposite to the first side of the polymer layer.

8. An electronic device (800), comprising:
- a gas sensor (100, 300, 500) comprising a layer of graphene (110) coupled to first (640, 650) and second (642, 652) electrodes;
- an electronic circuit (810) comprising:
a biasing circuit (705, 755) configured to bias the graphene layer by applying a voltage or a current to the first electrode; and
an analog-to-digital converter (730) configured to convert a voltage between the first electrode and the second electrode into a digital value; and
- a transmission circuit configured to wirelessly transmit the digital value via an antenna (820) of the electronic device.

9. The electronic device of claim 8, wherein the electronic circuit (810) is configured to be powered by energy harvesting of a received RF signal.

10. The electronic device of claim 8 or 9, wherein the gas sensor is implemented by the gas sensor (100, 300, 500) of any of claims 1 to 7.

11. A system (1000) comprising:
- the electronic device (800) according to one of claims 8 to 10; and
- a membrane (1010) attached to the electronic device and enclosing the gas sensor (100, 300, 500) of the electronic device, the membrane being suitable for being pierced by a straw for supplying a gas to the gas sensor.

12. A system (1100) comprising:
- a first compartment (1110) comprising an inlet (1140); and
- the electronic device (800) according to any one of claims 8 to 10, located inside the second compartment.

13. The system of claim 12, further comprising:
- an input valve (1145) at the interface between the first compartment and a second compartment (1120); and
- an output valve (1150) on one side of the second compartment.

14. A method of fabrication of a gas sensor, the method comprising:
- the deposition of metallic nanoparticles (115) on a layer of graphene (110); and
- the deposition of a first polymer layer (120) composed of parylene covering the graphene layer decorated with metallic nanoparticles.

15. A method of fabrication of a gas sensor, the method comprising:
- the deposition of metallic nanoparticles (115) on a first side of a layer of graphene (110);
- forming a first electrode (640, 650) in contact with the layer of graphene, for example by printing the first electrode in a first region of the first side of the layer of graphene (110) using conductive ink;
- the deposition of a first polymer layer (120) covering the first side of the layer of graphene;
- the deposition of metallic nanoparticles (315) on a second side of the layer of graphene, the second side being opposite to the first side;
- forming a second electrode (642, 652) in contact with the layer of graphene, for example by printing the second electrode in a second region of the second side of the layer of graphene (110) using conductive ink; and
- the deposition of a second polymer layer (320) covering the second side of the layer of graphene.
